# EUROPÄISCHE PATENTSCHRIFT

(11) **EP 4 401 889 B1**
(45) Veröffentlichungstag und Bekanntmachung des Hinweises auf die Patenterteilung: **09.07.2025**
(21) Anmeldenummer: 23715056.0
(22) Anmeldetag: 22.03.2023
(51) Int. Cl.: B05B 11/00

(54) **VORRICHTUNG ZUM PORTIONIERTEN AUSTRAGEN EINES FLÜSSIGEN MEDIUMS**
DEVICE FOR PORTIONED DISCHARGE OF A LIQUID MEDIUM
DISPOSITIF DE DÉCHARGE PORTIONNÉE D'UN MILIEU LIQUIDE

(30) Priorität: 22.03.2022 DE 102022000980
(43) Veröffentlichungstag der Anmeldung: 24.07.2024
(73) Patentinhaber: Novapacis UG (Haftungsbeschränkt), 40789 Monheim am Rhein (DE)
(72) Erfinder: MATANIC, Frank, 40547 Düsseldorf (DE)
(74) Vertreter: Janke Scholl Patentanwälte PartG mbB
(86) Internationale Anmeldenummer: PCT/EP2023/057375
(87) Internationale Veröffentlichungsnummer: WO 2023/180401

(56) Entgegenhaltungen:
- EP-B1- 3 072 597
- AU-B2- 2008 209 125
- GB-A- 2 140 509
- US-A- 4 286 736
- US-A- 6 070 763
- US-A1- 2011 073 679
- US-B2- 8 348 096

## Beschreibung

Die Erfindung betrifft eine Vorrichtung zum portionierten Austragen eines flüssigen, vorzugsweise therapeutischen Mediums nach dem Oberbegriff des Patentanspruchs 1.

Dosiervorrichtungen für Fluide, wie sie z. B. aus der EP 3 072 597 A1 bekannt sind, haben ein in einen Fluidbehälter hineinragendes Dosiergehäuse, in dem ein Kolben dichtend geführt ist. In dem Dosiergehäuse ist eine Druckkammer ausgebildet. Ein gegenüber dem Fluidbehälter wirkendes Ventil ist ausgebildet, bei Überdruck zu schließen und bei Unterdruck zu öffnen. Die Geometrie der Druckkammer definiert das Ausbringvolumen pro Hub. Der Kolben ist mit einer axialen Kolbenbohrung versehen und wird durch eine Rückstellfeder in Ruhelage gehalten. Bei Betätigung und axialer Bewegung des Kolbens wird zunächst das in der Druckkammer befindliche Fluid verdichtet und ein Überdruck erzeugt und dadurch das Ventil verschlossen. Sobald das Ventil verschlossen ist, wird bei weiterer axialer Kolbenbewegung und bei verschlossenem Ventil das Fluid durch die Kolbenbohrung hinaus gefördert.

Konventionelle Dosiervorrichtungen müssen häufig zu Beginn einige Male betätigt werden, um durch mehrfaches Pumpen zunächst Luft aus dem Dosiergehäuse zu verdrängen, bevor anschließend Fluid aus dem gespeicherten Vorrat angesaugt wird. Erst nach mehrmaliger Betätigung wird daher eine richtig portionierte Austragmenge erreicht. Zudem unterliegen manche Systeme einem Verlust der Austragmenge nach längerem Nichtgebrauch, wenn Fluid im Dosiergehäuse aufgrund Gravitation entweicht und in den Vorrat zurück gelangt.

Der Dosierverlust, verbunden mit erneuten Hüben der Dosiervorrichtung, ist zwar für Anwendungen in einigen technischen Bereichen unproblematisch. Bei Medizinprodukten und Arzneimitteln sind sie aber ein entscheidender Faktor bzw. Nachteil. So muss zum Beispiel in der pharmazeutischen Entwicklung einer Nasenspraypumpe neben der Dosiergenauigkeit pro Hub auch ein schnelles Ansprechen getestet werden, zudem müssen entsprechende Instruktionen auf den Produkten vorhanden sein. Eine Vorrichtung zum portionierten Austragen der gattungsgemäßen Art ist aus der DE 10 2009 017 459 A1 bekannt. Andere Austragsvorrichtungen sind aus der GB 2 140 509 A, US 4 286 736 A, DE 101 51 781 A1, der EP 1 194 246 A1 oder der EP 2 939 750 A1 bekannt. Die WO 2016/174031 A1 offenbart ein flexibles Dosiergehäuse, welches sich beim Betätigen und Ausbringen des Fluids zusammenzieht.

Der Erfindung liegt die **Aufgabe** zugrunde, bei kostengünstiger Herstellbarkeit mit einer geringen Anzahl von Komponenten ein bei der Betätigung der Vorrichtung verzögerungsfreies Ansprechen unter sofortigem Austragen der dosierten Austragmenge zu erzielen.

Zur Lösung dieser Aufgabe wird eine Vorrichtung zum portionierten Austragen eines flüssigen Mediums mit den Merkmalen des Patentanspruchs 1 vorgeschlagen. Die Vorrichtung umfasst
erste und zweite Gehäuseteile, die auf einer gemeinsamen Längsachse zwischen einer unbetätigten Ruhestellung und einer betätigten Endstellung zueinander längsbeweglich angeordnet sind,
einen zu dem ersten Gehäuseteil axial fest angeordneten Kolben,
einen zu dem zweiten Gehäuseteil axial fest angeordneten Druckstößel, der in Längsfluchtung zu dem Kolben angeordnet ist und sich aus einer geschlossenen Stirnfläche und einem Stößelmantel zusammensetzt,
eine von einer Innenwand des Kolbens umschlossene Dosierkavität, in die hinein der Druckstößel durch Betätigung der Vorrichtung absenkbar ist,
einen von der Dosierkavität ausgehenden Flüssigkeitskanal, welcher in Fluidverbindung mit einer ventilgesteuerten Austragöffnung in dem ersten Gehäuseteil steht.

Gekennzeichnet ist die erfindungsgemäße Vorrichtung durch einen an der Innenwand des Kolbens ausgebildeten, inneren Dichtabschnitt, gegen den bei teilweise und bei vollständig in die Dosierkavität hinein abgesenktem Druckstößel ein außen an dem Stößelmantel ausgebildeter, äußerer Dichtabschnitt spaltfrei anliegt.

Die Betätigung der Vorrichtung lässt sich in einzelne Phasen gliedern. Die erste Phase ist der noch unbetätigte Ruhe- oder Ausgangszustand. In diesem Zustand besteht erfindungsgemäß kein Kontakt und damit auch keine gegenseitige Abdichtung zwischen dem Kolben und dem Druckstößel. Daher kann die z. B. therapeutische Flüssigkeit ungehindert in die Dosierkavität einströmen und diese füllen.

In der zweiten und dritten Phase hingegen, nämlich bei teilweise in die Dosierkavität hinein abgesenktem Druckstößel und bei vollständig abgesenktem Druckstößel, liegt der innere Dichtabschnitt spaltfrei an dem gegenüberliegenden äußeren Dichtabschnitt an, so dass die Dosierkavität ab der zweiten Phase von dem Flüssigkeitsvorrat in dem umgebenden Speicherraum getrennt ist. In der dritten Phase erfolgt dann ein Druck auf das so abgetrennte Medium, wodurch dieses in Richtung Austragöffnung gepresst wird und diese verlässt.

Vorteilhafte, aber nicht obligatorische Ausgestaltungen der erfindungsgemäßen Vorrichtung sind in den einzelnen Unteransprüchen angegeben.

Mit einer ersten Ausgestaltung ist vorgesehen, dass sich der Flüssigkeitskanal in dem Kolben befindet, indem der Flüssigkeitskanal, vom Boden der Dosierkavität ausgehend, zunächst entlang des Kolbens führt.

Der das Medium bevorratende Speicherraum ist vorzugsweise Bestandteil des zweiten Gehäuseteils. Die ventilgesteuerte Austragöffnung befindet sich vorzugsweise an dem ersten Gehäuseteil.

Von Vorteil für die Funktion der Dosierkavität ist, wenn sich die Anordnung aus innerem und äußerem Dichtabschnitt in dem Speicherraum befindet, und von dem Speicherraum umgeben ist.

Von Vorteil für die Funktion der Dosierkavität ist außerdem, wenn der Druckstößel, der Stirnfläche abgewandt, an einem Stößelträger angeordnet ist, wobei sich der Stößelträger in dem Speicherraum befindet und mit mehreren für das Medium durchströmbaren Öffnungen versehen ist. Die Öffnungen können über den Umfang des Stößelträgers verteilt angeordnet sein. Der Stößelträger bildet in diesem Fall einen Bewegungsraum für den darin axial beweglich angeordneten Kolben.

Die Innenwand der Dosierkavität kann auf dem überwiegenden Teil ihrer Länge als ein kreiszylindrischer Längsabschnitt gestaltet sein, an den sich, zu dem Druckstößel hin, ein sich konisch verjüngender Längsabschnitt anschließt.

Ferner vorgeschlagen wird ein im Übergang zwischen der Dosierkavität und dem Flüssigkeitskanal angeordnetes Ventil. Das Ventil ist dazu ausgebildet, nur zu dem Flüssigkeitskanal hin zu öffnen. Das Ventil ermöglicht in einer Verdrängungsphase eine Verdichtung in der Dosierkavität. Erst bei Erreichen einer Austragphase öffnet das Ventil und gibt den Flüssigkeitskanal für das Hindurchströmen des Mediums frei, wobei das Ventil, nach Abschluss des Austrags, Leckagen, Rückfluss oder Kontamination des in dem Speicherraum bevorrateten Mediums verhindert.

Ausführungsbeispiele der Vorrichtung werden nachfolgend in weiteren Einzelheiten und unter Bezug auf die Zeichnungen erläutert. Darin zeigen:
- Fig. 1: eine erste Ausführungsform der Vorrichtung zum portionierten Austragen eines flüssigen Mediums, und zwar in noch unbetätigter Ruhestellung bzw. Ausgangsstellung;
- Fig. 2: dieselbe Vorrichtung in einer Zwischenphase der Betätigung, in der es erstmals zu einer Separierung der Dosierkavität von dem Speicherraum kommt;
- Fig. 3: dieselbe Vorrichtung in der Endstellung der Betätigung, d. h. am Ende der Ausbringungsphase;
- Fig. 4: eine zweite Ausführungsform der Vorrichtung zum portionierten Austragen eines flüssigen Mediums, und zwar in der Zwischenphase, wie sie in Fig. 2 für die erste Ausführungsform wiedergegeben ist; und
- Fig. 5: eine dritte Ausführungsform der Vorrichtung zum portionierten Austragen eines flüssigen Mediums, und zwar wiederum in der Zwischenphase, wie sie in Fig. 2 für die erste Ausführungsform wiedergegeben ist.

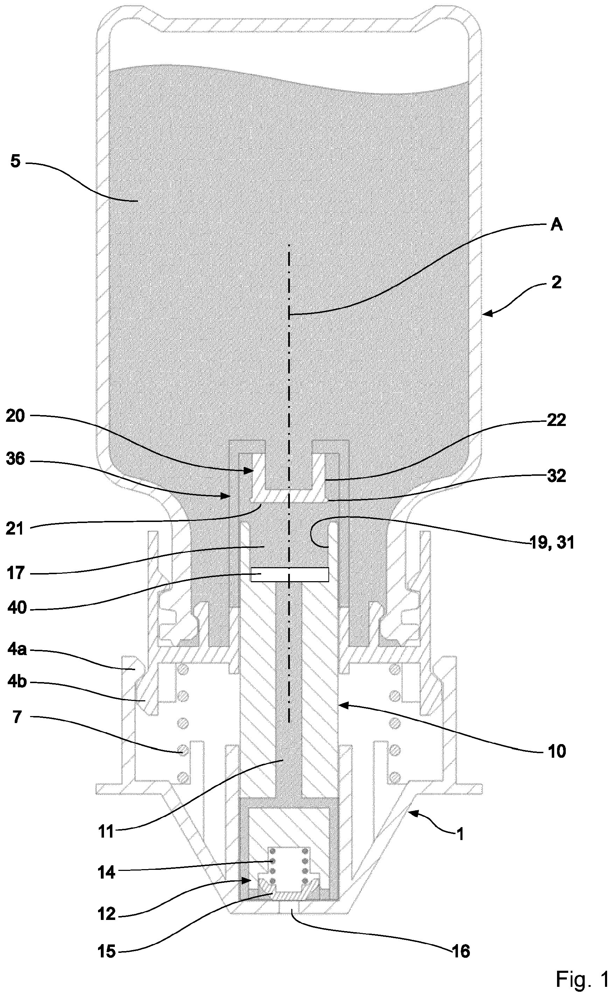

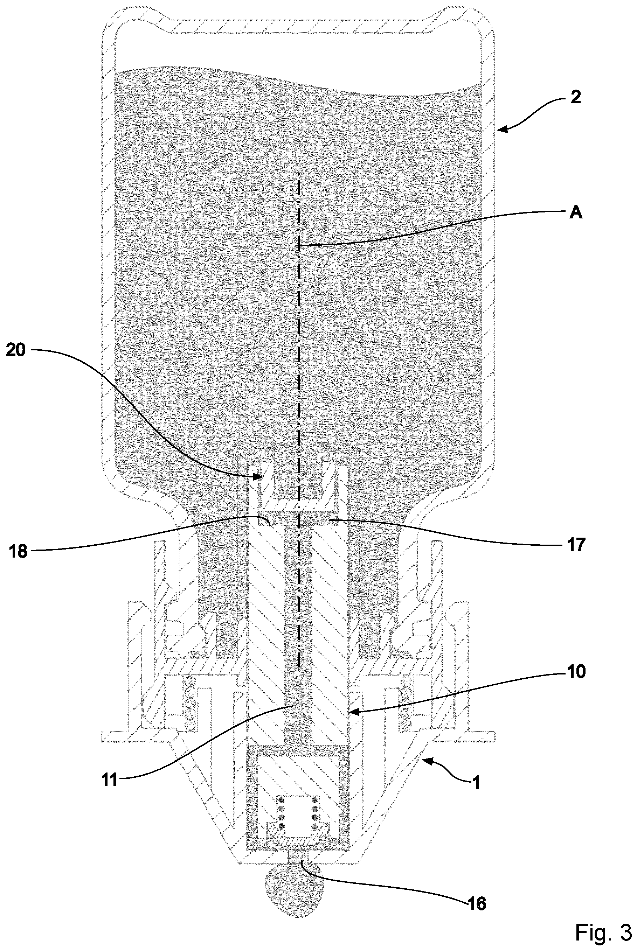

Die in den Figuren 1 bis 3 in einer ersten Ausführungsform dargestellte Vorrichtung dient dem portionierten Austragen eines flüssigen Mediums und konkret einer therapeutischen Flüssigkeit, zum Beispiel von Nasentropfen oder von Augentropfen. Ebenso lassen sich mit der Vorrichtung auch andere Medien und Medien für andere Zwecke portioniert, d. h. in definierten und reproduzierbaren Volumeneinheiten, austragen. Die Vorrichtung umfasst ein erstes Gehäuseteil 1 und ein zweites Gehäuseteil 2, die auf einer gemeinsamen Längsachse A zwischen einer unbetätigten Ruhestellung (Fig. 1) und einer betätigten Endstellung (Fig. 3) zueinander längsbeweglich angeordnet sind. Die Betätigung durch den Anwender erfolgt üblicherweise per Hand, indem der Daumen gegen das Ende des zweiten Gehäuseteils 2 drückt, und sich zugleich zwei Finger an dem ersten Gehäuseteil 1 abstützen und den Gegendruck aufnehmen.

Die Gehäuseteile 1, 2 können, was die Handhabung erleichtert, rotationssymmetrisch gestaltet sein. Sie sind mit einseitig arbeitenden Verriegelungsstrukturen 4a, 4b versehen. Diese lassen zwar das Zusammenschieben der beiden Gehäuseteile 1, 2 unter Abgabe, d.h. unter Austrag einer dosierten Flüssigkeitsmenge zu, nicht jedoch ein voneinander Lösen der beiden Gehäuseteile 1, 2. Eine Feder 7 zwischen den Gehäuseteilen 1, 2 sorgt dafür, dass diese bei Nichtbetätigung zurück in die Ausgangs- bzw. Ruhestellung geschoben werden, d. h. bis zur gegenseitigen Anlage der Verriegelungsstrukturen 4a, 4b.

Bestandteil des zweiten Gehäuseteils 2 ist ein Speicherraum 5, in dem das zu verabreichende flüssige Medium bevorratet ist. Die Menge reicht aus, eine Vielzahl von Austrägen durchzuführen. Der Speicherraum 5 kann, je nach Gestaltung der Gehäuseteile 1, 2, auch Raumanteile umfassen, die sich innerhalb des erste Gehäuseteils 1 befinden.

Ein Kolben 10 ist Bestandteil des ersten Gehäuseteils 1 und ist daher axial fest zu dem ersten Gehäuseteil angeordnet. Der Kolben 10 hat die Form eines Stempels und erstreckt sich auf der Längsachse A. Er ist in seinem Zentrum mit einem Flüssigkeitskanal 11 versehen, der zu einer in dem ersten Gehäuseteil 1 angeordneten Ventileinheit 12 führt. In den Zeichnungen ist, aus Gründen der Darstellung, der Kanalquerschnitt des Flüssigkeitskanals 11 übertrieben groß wiedergegeben.

Bestandteil der Ventileinheit 12 ist ein durch eine Schließfeder 14 in Schließrichtung belasteter und druckabhängig arbeitender Ventilkörper 15. In seiner in Fig. 1 wiedergegebenen Schließstellung verschließt der Ventilkörper 15 eine Austragöffnung 16, die sich am Ende des ersten Gehäuseteils 1 befindet.

Der Ventilkörper 15 ist dazu ausgebildet, nur bei hinreichend hohem Druck des flüssigen Mediums entgegen des Federdrucks der Schließfeder 14 zu öffnen, und so die Austragöffnung 16 freizugeben. Auf diese Weise gelangt Flüssigkeit von dem Flüssigkeitskanal 11 und vorzugsweise unter teilweiser Umströmung der Ventileinheit 12 zu der Austragöffnung 16, und spritzt aus dieser heraus.

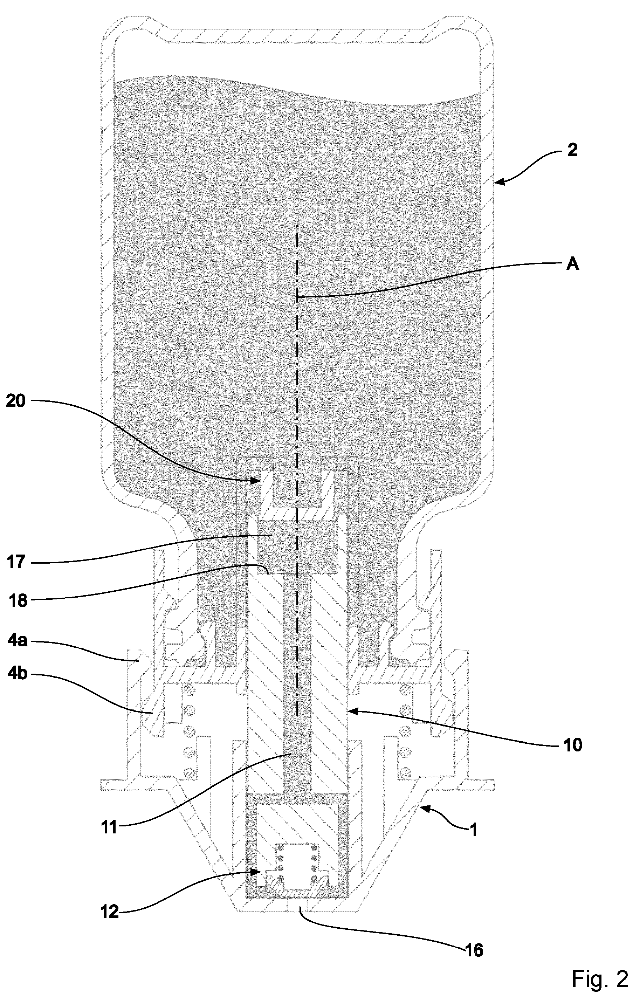

An seinem anderen Ende endet der Flüssigkeitskanal 11 in einer Dosierkavität 17. Bei der Dosierkavität 17 handelt es sich um einen topfförmigen, im Wesentlichen zylindrischen Raum am Ende des Kolbens 10 und auf der Längsachse A. Umfangsmäßig ist die Dosierkavität 17 von einer Innenwandung 19 des Kolbens 10 umschlossen. Die Innenwandung 19 ist vollständig verschlossen. An ihrem einen axialen Ende weist die Dosierkavität 17 einen Boden 18 auf (Fig. 2), von dem der Flüssigkeitskanal 11 ausgeht. An ihrem anderen Ende ist die Dosierkavität 17 offen.

Ein Druckstößel 20 ist Bestandteil des zweiten Gehäuseteils 2 und ist daher axial fest zu dem zweiten Gehäuseteil angeordnet. Auch der Druckstößel 20 erstreckt sich auf der Längsachse A, und daher in Längsfluchtung zu dem Kolben 10. Auch der Druckstößel 20 hat die Form eines Stempels und setzt sich aus einer Stirnfläche 21, die der Dosierkavität 17 zugewandt ist, und aus einem Stößelmantel 22 zusammen. Die Stirnfläche 21 ist als eine geschlossene Druckfläche ausgebildet. Der Stößelmantel 22 ist beim gezeigten Ausführungsbeispiel ebenfalls geschlossen, kann jedoch auch mit Öffnungen versehen sein.

Der Druckstößel 20 des zweiten Gehäuseteils 2 ist im Zusammenwirken mit dem Kolben 10 des ersten Gehäuseteils 1 dazu bestimmt, durch und während der Betätigung der Vorrichtung in die Dosierkavität 17 hinein abgesenkt zu werden, und hierbei eine bestimmte Flüssigkeitsmenge zunächst in der Dosierkavität zu portionieren und anschließend den für das Austragen der Flüssigkeitsmenge erforderlichen Druck aufzubringen. Die hierbei ausgeführte Relativbewegung erfolgt auf der Längsachse A.

Für beide genannten Wirkungen ist eine phasenweise Abdichtung zwischen der Dosierkavität 17 und dem Druckstößel 20 erforderlich. Hierfür bildet die die Dosierkavität 17 umschließende Innenwand 19 des Kolbens 10 einen inneren Dichtabschnitt 31. Gegen den inneren Dichtabschnitt 31 liegt, bei zumindest teilweise in die Dosierkavität 17 hinein abgesenktem Druckstößel 20 (Fig. 2) und ebenso bei vollständig in die Dosierkavität 17 hinein abgesenktem Druckstößel 20 (Fig. 3), ein gegenüberliegender Dichtabschnitt 32 spaltfrei an, welcher sich außen an dem Stößelmantel 22 befindet. Beim Ausführungsbeispiel ist dieser äußere Dichtabschnitt 32 ein Dichtwulst, welcher in Höhe der Stirnfläche 22 ringförmig um den Stößelmantel 22 führt.

Die Fig. 2 zeigt hierbei den Zustand des erstmaligen Kontakts zwischen dem Kolben 10 und dem Druckstößel 20, wobei dieser Kontakt an den Dichtabschnitten 31, 32 erfolgt. Von diesem Zeitpunkt an ist die Portionierung in der Dosierkavität 17 abgeschlossen. Weitere Flüssigkeit kann nicht mehr in die Dosierkavität gelangen. Ab diesem Zeitpunkt beginnt die Druckphase mit Austrag der dosierten Flüssigkeitsmenge. Deren Abschluss zeigt die Fig. 3.

Demgegenüber zeigt die Fig. 1 für den noch nicht betätigten Ruhe- oder Ausgangszustand, dass in diesem Zustand noch keine Abdichtung zwischen der Innenwand 19 des Kolbens 10 und dem Druckstößel 20 besteht, da in diesem Zustand der Kolben 10 noch in einem axialen Abstand zu dem Druckstößel 20 angeordnet ist, daher auch die Dichtabschnitte 31, 32 noch keinen Kontakt haben, und daher therapeutische Flüssigkeit ungehindert in die Dosierkavität 17 gelangen kann. Insbesondere befindet sich daher die Anordnung aus den inneren und äußeren Dichtabschnitten 31, 32 in dem Speicherraum 5 und ist von dem Speicherraum 5 umgeben, was zu einer zuverlässigen Zuströmung von Flüssigkeit aus dem Speicherraum in die Dosierkavität 17 führt.

Zu dieser Zuströmung trägt auch bei, dass der Druckstößel 20 an einem Stößelträger 36 angeordnet ist, der sich in dem Speicherraum 5 befindet und mit mehreren für das Medium durchströmbaren Öffnungen 37 versehen ist. Die Öffnungen 37 sind über den Umfang des Stößelträgers 36 verteilt angeordnet, vorzugsweise gleichmäßig verteilt. Das Innere des Stößelträgers 36 bildet einen Bewegungsraum für den darin axial beweglichen Kolben 10.

Um in der in Fig. 2 wiedergegebenen Zwischenphase, in der es erstmals zum Kontakt der Dichtabschnitte 31, 32 kommt, die mechanische Beanspruchung in den Kantenbereichen gering zu halten, ist die Innenwand 19 der Dosierkavität 17 auf dem überwiegenden Teil ihrer Länge als ein kreiszylindrischer Längsabschnitt gestaltet, an den sich zu dem Druckstößel 20 hin ein kürzerer, sich konisch verjüngender Längsabschnitt anschließt.

Die Fig. 1 zeigt zusätzlich ein Ventil 40, welches zwischen der Dosierkavität 17 und dem Flüssigkeitskanal 11 angeordnet ist und beim Ausführungsbeispiel am Boden 18 der Kavität 17 platziert ist. In den übrigen Figuren der Zeichnung ist das Ventil 40 nicht ausdrücklich dargestellt. Das Ventil 40 ermöglicht in der Verdrängungsphase eine Verdichtung in der Dosierkavität 17. Es öffnet erst bei Erreichen der Austragphase und gibt dann den Flüssigkeitskanal 11 für das Hindurchströmen der dosierten Flüssigkeitsmenge frei. Nach Abschluss des Flüssigkeitsaustrags verhindert das Ventil 40 ein Rückströmen oder eine Kontamination des Restvolumens in der Kolbenbohrung. Die Dosiervorrichtung kann also konstruktiv und funktionell das Füllgut im Behältnis gegen Kontamination schützen.

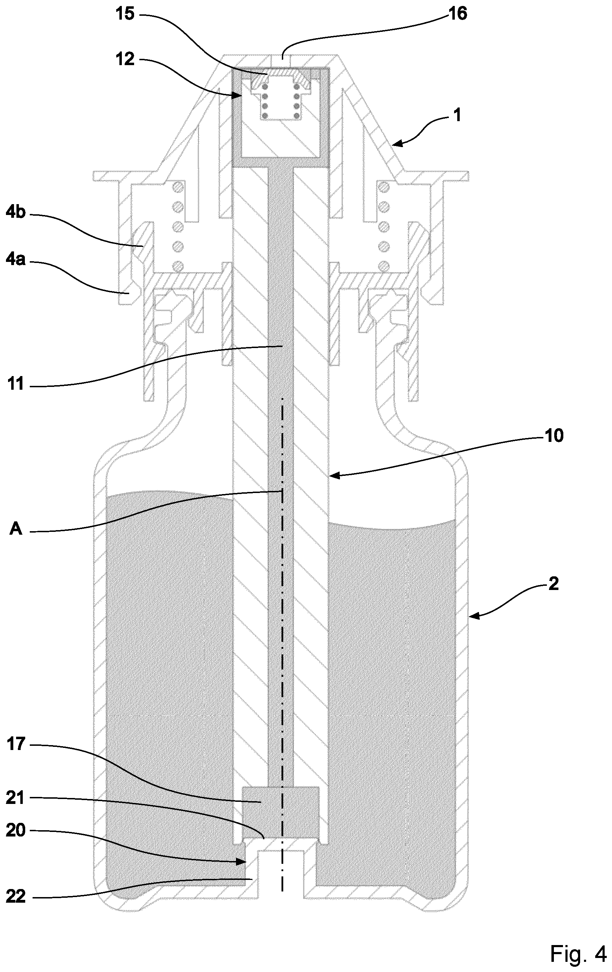

Die Fig. 4 zeigt eine zweite Ausführungsform der Vorrichtung. Dargestellt ist nur die Zwischenphase, wie sie in Fig. 2 für die erste Ausführungsform wiedergegeben ist. Bei der Handhabung der zweiten Ausführungsform ist die Anordnung der Gehäuseteile umgekehrt zu jener nach den Figuren 1 bis 3. Das erste Gehäuseteil 1 mit dem Kolben 10 und der Austragöffnung 16 befindet sich hier oben, und das zweite Gehäuseteil 2 mit dem Druckstößel 20 unten. Durch die hier nach oben gerichtete Austragöffnung 16 eignet sich diese Ausführungsform z. B. zur Verabreichung von Nasentropfen, wohingegen sich die erste Ausführungsform auf dem Gebiet der Medizin eher für die Verabreichung von Augentropfen eignet.

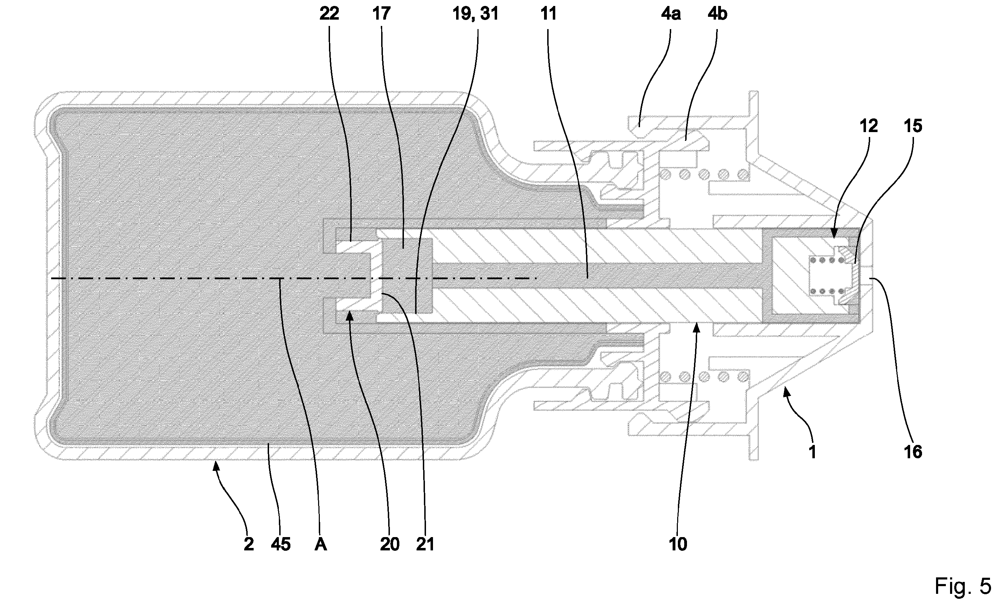

Die Fig. 5 zeigt eine dritte Ausführungsform. Dargestellt ist wiederum nur die Zwischenphase, wie sie in Fig. 2 für die erste Ausführungsform und in Fig. 4 für die zweite Ausführungsform wiedergegeben ist.

Bei der Handhabung der dritten Ausführungsform ist die Anordnung der Gehäuseteile 1, 2 ohne größere Bedeutung. Als Beispiel dargestellt ist hier die Anordnung mit horizontaler Längsachse A, so dass auch die Austragöffnung 16 für eine entsprechende Applikation horizontal gerichtet ist.

Bei Fig. 5 ist der Speicherraum 5 für das zu verabreichende flüssige Medium wiederum von dem festen Gehäusematerial des zweiten Gehäuseteils 2 umschlossen, und zusätzlich von einer inneren Auskleidung 45 in Gestalt eines Beutels. Die Auskleidung 45 besteht aus flexiblem und für Flüssigkeit undurchlässigem Material. Sie liegt anfangs gegen die Innenwand des Gehäuseteils 2 an, ist mit dieser allerdings nicht verbunden und ferner so ausgebildet und abgedichtet, dass die gespeicherte Flüssigkeit vollständig und luftdicht eingeschlossen ist. Nimmt das Flüssigkeitsvolumen infolge des wiederholten Austragens sukzessive ab, führt dies zu einer entsprechenden Verkleinerung der Auskleidung 45, indem sich der Beutel zusammenzieht. Der so umhüllte (Rest)Speicherraum 5 bleibt jedoch stets vollständig mit flüssigem Medium gefüllt, und damit auch der Bereich rund um die Dosierkavität 17.

### Bezugszeichen

- 1: erstes Gehäuseteil
- 2: zweites Gehäuseteil
- 4a: Verriegelungsstruktur
- 4b: Verriegelungsstruktur
- 5: Speicherraum
- 7: Feder
- 10: Kolben
- 11: Flüssigkeitskanal
- 12: Ventileinheit
- 14: Schließfeder
- 15: Ventilkörper
- 16: Austragöffnung
- 17: Dosierkavität
- 18: Boden
- 19: Innenwand
- 20: Druckstößel
- 21: Stirnfläche
- 22: Stößelmantel
- 31: innerer Dichtabschnitt
- 32: äußerer Dichtabschnitt
- 36: Stößelträger
- 40: Ventil
- 45: Auskleidung

- A: Längsachse

## Patentansprüche

1. Vorrichtung zum portionierten Austragen eines flüssigen, vorzugsweise therapeutischen Mediums, mit
ersten und zweiten Gehäuseteilen (1, 2), die auf einer gemeinsamen Längsachse (A) zwischen einer unbetätigten Ruhestellung und einer betätigten Endstellung zueinander längsbeweglich angeordnet sind,
einem zu dem ersten Gehäuseteil (1) axial fest angeordneten Kolben (10),
einem zu dem zweiten Gehäuseteil (2) axial fest angeordneten Druckstößel (20), der in Längsfluchtung zu dem Kolben (10) angeordnet ist und sich aus einer geschlossenen Stirnfläche (21) und einem Stößelmantel (22) zusammensetzt,
einer von einer Innenwand (19) des Kolbens (10) umschlossenen Dosierkavität (17), in die hinein der Druckstößel (20) durch Betätigung der Vorrichtung absenkbar ist, und einem von der Dosierkavität (17) ausgehenden Flüssigkeitskanal (11), welcher in Fluidverbindung mit einer ventilgesteuerten Austragöffnung (16) in dem ersten Gehäuseteil (1) steht,
**gekennzeichnet durch** einen an der Innenwand (19) des Kolbens (10) ausgebildeten, inneren Dichtabschnitt (31), gegen den bei teilweise und bei vollständig in die Dosierkavität (17) hinein abgesenktem Druckstößel (20) ein außen an dem Stößelmantel (22) ausgebildeter, äußerer Dichtabschnitt (32) spaltfrei anliegt, wobei die Betätigung der Vorrichtung in einzelne Phasen gegliedert ist, wobei eine erste Phase der noch unbetätigte Ruhe- oder Ausgangszustand ist, in dem kein Kontakt und damit auch keine gegenseitige Abdichtung zwischen dem Kolben (10) und dem Druckstößel (20) besteht, wobei therapeutische Flüssigkeit in der ersten Phase ungehindert aus einem Flüssigkeitsvorrat eines die Dosierkavität (17) umgebenden Speicherraums (5) in die Dosierkavität (17) einströmen und diese füllen kann, wobei in einer zweiten und dritten Phase, bei teilweise in die Dosierkavität (17) hinein abgesenktem Druckstößel (20) und bei vollständig abgesenktem Druckstößel (20), der innere Dichtabschnitt (31) spaltfrei an dem gegenüberliegenden äußeren Dichtabschnitt (32) anliegt, so dass die Dosierkavität (17) ab der zweiten Phase von dem Flüssigkeitsvorrat des umgebenden Speicherraums (5) getrennt ist und in der dritten Phase ein Druck auf das so abgetrennte Medium erfolgt, wodurch dieses in Richtung der Austragöffnung (16) gepresst wird und diese verlässt.

2. Vorrichtung nach Anspruch 1, **dadurch gekennzeichnet, dass** der Flüssigkeitskanal (11), vom Boden (18) der Dosierkavität (17) ausgehend, zunächst entlang des Kolbens (10) führt.

3. Vorrichtung nach Anspruch 1 oder 2, **dadurch gekennzeichnet, dass** sich die ventilgesteuerte Austragöffnung (16) an dem ersten Gehäuseteil (1) befindet.

4. Vorrichtung nach einem der Ansprüche 1 bis 3, **dadurch gekennzeichnet, dass** Bestandteil des zweiten Gehäuseteils (2) ein das Medium bevorratender Speicherraum (5) ist.

5. Vorrichtung nach Anspruch 4, **dadurch gekennzeichnet, dass** sich die Anordnung aus den inneren und äußeren Dichtabschnitten (31, 32) in dem Speicherraum (5) befindet und von dem Speicherraum (5) umgeben ist.

6. Vorrichtung nach Anspruch 4 oder 5, **dadurch gekennzeichnet, dass** der Druckstößel (20), der Stirnfläche (21) abgewandt, an einem Stößelträger (36) angeordnet ist, und dass sich der Stößelträger (36) in dem Speicherraum (5) befindet und mit mehreren für das Medium durchströmbaren Öffnungen versehen ist.

7. Vorrichtung nach Anspruch 6, **dadurch gekennzeichnet, dass** die Öffnungen über den Umfang des Stößelträgers (36) verteilt angeordnet sind, vorzugsweise gleichmäßig verteilt.

8. Vorrichtung nach Anspruch 6 oder 7, **dadurch gekennzeichnet, dass** das Innere des Stößelträgers (36) als ein Bewegungsraum für den darin axial beweglich angeordneten Kolben (10) ausgebildet ist.

9. Vorrichtung nach einem der vorangehenden Ansprüche, **dadurch gekennzeichnet, dass** die Innenwand (19) der Dosierkavität (17) auf dem überwiegenden Teil ihrer Länge als ein kreiszylindrischer Längsabschnitt gestaltet ist, an den sich, zu dem Druckstößel (20) hin, ein sich konisch verjüngender Längsabschnitt anschließt.

10. Vorrichtung nach einem der vorangehenden Ansprüche, **gekennzeichnet durch** ein im Übergang zwischen der Dosierkavität (17) und dem Flüssigkeitskanal (11) angeordnetes Ventil (40).

11. Vorrichtung nach Anspruch 10, **dadurch gekennzeichnet, dass** das Ventil (40) in einer Verdrängungsphase eine Verdichtung in der Dosierkavität (17) ermöglicht und erst bei Erreichen einer Austragphase öffnet und den Flüssigkeitskanal (11) für das Hindurchströmen des Mediums freigibt, und dass das Ventil (40), nach Abschluss des Austrags, Leckagen, Rückfluss oder Kontamination des in dem Speicherraum bevorrateten Mediums verhindert.

## Claims

1. Device for the portioned dispensing of a liquid, preferably therapeutic, medium, having
first and second housing parts (1, 2) which are arranged to be longitudinally movable relative to one another on a common longitudinal axis (A) between an unactuated rest position and an actuated end position,
a piston (10) arranged axially fixed to the first housing part (1),
a pressure plunger (20) arranged axially fixed to the second housing part (2), which is arranged in longitudinal alignment with the piston (10) and is composed of a closed end face (21) and a plunger casing (22),
a dosing cavity (17) which is enclosed by an inner wall (19) of the piston (10) and into which the pressure plunger (20) can be lowered by actuation of the device, and
a liquid channel (11) which extends from the dosing cavity (17) and is in fluid communication with a valve-controlled discharge opening (16) in the first housing part (1),
**characterized by** an inner sealing section (31) formed on the inner wall (19) of the piston (10), against which, when the pressure plunger (20) is partially or completely lowered into the dosing cavity (17), an outer sealing section (32) formed on the outside of the plunger casing (22) bears against it without any gap, wherein the actuation of the device is divided into individual phases, wherein a first phase is the still unactuated rest or initial state, in which there is no contact and therefore no mutual sealing between the piston (10) and the pressure plunger (20), therapeutic fluid being able to flow unhindered from a fluid supply in a storage space (5) surrounding the dosing cavity (17) into the dosing cavity (17) and fill the latter in the first phase, in a second and third phase, with the pressure plunger (20) partially lowered into the dosing cavity (17) and with the pressure plunger (20) fully lowered, the inner sealing section (31) rests against the opposite outer sealing section (32) without any gaps, so that the dosing cavity (17) is separated from the fluid supply of the surrounding storage space (5) from the second phase onwards and, in the third phase, pressure is applied to the medium thus separated, causing it to be pressed toward the discharge opening (16) and to leave it.

2. Device according to claim 1, **characterized in that** the liquid channel (11), starting from the base (18) of the dosing cavity (17), initially runs along the piston (10).

3. Device according to claim 1 or 2, **characterized in that** the valve-controlled discharge opening (16) is located on the first housing part (1).

4. Device according to one of claims 1 to 3, **characterized in that** part of the second housing part (2) is a storage space (5) storing the medium.

5. Device according to claim 4, **characterized in that** the arrangement of the inner and outer sealing sections (31, 32) is located in the storage space (5) and is surrounded by the storage space (5).

6. Device according to claim 4 or 5, **characterized in that** the pressure plunger (20) is arranged on a plunger carrier (36) facing away from the end face (21), and **in that** the plunger carrier (36) is located in the storage space (5) and is provided with a plurality of openings through which the medium can flow.

7. Device according to claim 6, **characterized in that** the openings are arranged distributed around the circumference of the pressure plunger carrier (36), preferably evenly distributed.

8. Device according to claim 6 or 7, **characterized in that** the interior of the pressure plunger carrier (36) is designed as a movement space for the piston (10) arranged axially movably therein.

9. Device according to one of the preceding claims, **characterized in that** the inner wall (19) of the dosing cavity (17) is designed over the predominant part of its length as a circular cylindrical longitudinal section which is adjoined by a conically tapering longitudinal section towards the pressure plunger (20).

10. Device according to one of the preceding claims, **characterized by** a valve (40) arranged in the transition between the dosing cavity (17) and the liquid channel (11).

11. Device according to claim 10, **characterized in that** the valve (40) enables compression in the dosing cavity (17) in a displacement phase and only opens when a discharge phase is reached and releases the liquid channel (11) for the medium to flow through, and **in that** the valve (40) prevents leakage, backflow or contamination of the medium stored in the storage space after the discharge has been completed.

## Revendications

1. Dispositif de distribution par portions d'un milieu fluide, de préférence thérapeutique, comportant
des première et deuxième parties de boîtier (1, 2), qui sont disposées de façon à pouvoir se déplacer longitudinalement l'une par rapport à l'autre sur un axe longitudinal commun (A) entre une position de repos, non actionnée, et une position finale, actionnée,
un piston (10) disposé axialement de façon fixe par rapport à la première partie de boîtier (1),
un poussoir (20) disposé axialement de façon fixe par rapport à la deuxième partie de boîtier (2), qui est disposé en alignement longitudinal par rapport au piston (10) et qui se compose d'une surface avant fermée (21) et d'une enveloppe de poussoir (22),
une cavité de dosage (17) entourée par une paroi intérieure (19) du piston (10), dans laquelle le poussoir (20) peut être abaissé par actionnement du dispositif, et
un canal de fluide (11) partant de la cavité de dosage (17) et qui se trouve en communication fluidique avec un orifice de distribution (16), commandé par une soupape, dans la première partie de boîtier (1),
**caractérisé par** un segment d'étanchéité interne (31) réalisé au niveau de la paroi intérieure (19) du piston (10), contre laquelle repose sans espace, lorsque le poussoir (20) est partiellement et complètement abaissé dans la cavité de dosage (17), un segment d'étanchéité externe (32) réalisé à l'extérieur au niveau de l'enveloppe de poussoir (22), l'actionnement du dispositif étant divisé en différentes phases, une première phase étant l'état de repos ou l'état initial non encore actionné, dans lequel il n'existe aucun contact et par conséquent aucune étanchéité mutuelle entre le piston (10) et le poussoir (20), un fluide thérapeutique pouvant, dans la première phase, s'écouler librement depuis un réservoir de fluide d'un espace de stockage (5) entourant la cavité de dosage (17) dans la cavité de dosage (17) et la remplir, et le segment d'étanchéité interne (31), lors d'une deuxième et d'une troisième phase, lorsque le poussoir (20) est partiellement abaissé dans la cavité de dosage (17) et lorsque le poussoir (20) est complètement abaissé, reposant sans espace contre le segment d'étanchéité externe (32) opposé, de sorte que la cavité de dosage (17), à partir de la deuxième phase, est séparée du réservoir de fluide de l'espace de stockage (5) environnant et que, lors de la troisième phase, s'exerce une pression sur le milieu ainsi séparé, celui-ci étant comprimé en direction de l'orifice de distribution (16) et quittant celui-ci.

2. Dispositif selon la revendication 1, **caractérisé en ce que** le canal de fluide (11), en partant du fond (18) de la cavité de dosage (17), s'étend d'abord le long du piston (10).

3. Dispositif selon la revendication 1 ou 2, **caractérisé en ce que** l'orifice de distribution (16) commandé par soupape se trouve au niveau de la première partie de boîtier (1).

4. Dispositif selon l'une des revendications 1 à 3, **caractérisé en ce qu'**un espace de stockage (5) permettant l'approvisionnement en fluide est un composant de la deuxième partie de boîtier (2).

5. Dispositif selon la revendication 4, **caractérisé en ce que** l'ensemble des segments d'étanchéité interne et externe (31, 32) est situé dans l'espace de stockage (5) et est entouré par l'espace de stockage (5).

6. Dispositif selon la revendication 4 ou 5, **caractérisé en ce que** le poussoir (20) est disposé au niveau d'un support de poussoir (36) dirigé à l'opposé de la surface avant (21), et **en ce que** le support de poussoir (36) se trouve dans l'espace de stockage (5) et est pourvu de plusieurs ouvertures pouvant être traversées par le fluide.

7. Dispositif selon la revendication 6, **caractérisé en ce que** les ouvertures sont réparties sur la circonférence du support de poussoir (36), de préférence uniformément réparties.

8. Dispositif selon la revendication 6 ou 7, **caractérisé en ce que** l'intérieur du support de poussoir (36) est réalisé comme un espace de déplacement pour le piston (10) disposé de façon à pouvoir se déplacer axialement à l'intérieur de celui-ci.

9. Dispositif selon l'une des revendications précédentes, **caractérisé en ce que** la paroi intérieure (19) de la cavité de dosage (17) est réalisée, sur la majeure partie de sa longueur, en tant que segment longitudinal cylindrique circulaire au niveau duquel se raccorde, en direction du poussoir (20), un segment longitudinal qui se rétrécit en cône.

10. Dispositif selon l'une des revendications précédentes, **caractérisé par** une soupape (40) disposée dans le passage entre la cavité de dosage (17) et le canal de fluide (11).

11. Dispositif selon la revendication 10, **caractérisé en ce que** la soupape (40) permet, dans une phase de déplacement, une compression dans la cavité de dosage (17) et ne s'ouvre que lorsqu'une phase de distribution est atteinte et autorise le passage du milieu dans le canal de fluide (11), et **en ce que** la soupape (40), une fois la distribution terminée, empêche les fuites, le reflux ou la contamination du milieu stocké dans l'espace de stockage.
